# EUROPEAN PATENT SPECIFICATION

(11) **EP 3 665 299 B1**
(45) Date of publication and mention of the grant of the patent: **21.07.2021**
(21) Application number: 18758681.3
(22) Date of filing: 07.08.2018
(51) Int. Cl.: C12Q 1/04, C12Q 1/10, C12Q 1/25, C12Q 1/34

(54) **DETECTION OF CARBAPENEMASE-PRODUCING ENTEROBACTERIALES PSEUDOMONAS AND ACINETOBACTER SPECIES USING A CHROMOGEN**
NACHWEIS VON CARBAPENEMASE-PRODUZIERENDEN ENTEROBAKTERIEN PSEUDOMONAS- UND ACINETOBACTER-ARTEN UNTER VERWENDUNG EINES CHROMOGENS
DÉTECTION DES ENTÉROBACTÉRIENS PRODUCTEURS DE CARBAPÉNÉMASES CHEZ LES ESPÈCES DE PSEUDOMONAS ET D'ACINETOBACTER À L'AIDE D'UN CHROMOGÈNE

(30) Priority: 07.08.2017 GB 201712671
(43) Date of publication of application: 17.06.2020
(73) Proprietor: Mast Group Limited, Liverpool, Merseyside L20 1EA (GB)
(72) Inventor: HOBSON, Jonathan Anthony, Liverpool Merseyside L25 3PE (GB); COLEBORN, Matilda May, Liverpool Merseyside L7 8SW (GB); DAVIES, Mya, Liverpool Merseyside L28 3QB (GB); ANYAKWO, Andrew Chuka, Bootle Merseyside L20 4PN (GB)
(74) Representative: Forrest, Stuart
(86) International application number: PCT/GB2018/052255
(87) International publication number: WO 2019/030519

(56) References cited:
- EP-A1- 1 325 923
- EP-A1- 1 557 473
- WO-A1-2009/051838
- WO-A1-2017/089823
- FR-A1- 2 956 866

## Description

### FIELD OF INVENTION

The present invention relates to a method for detecting the presence of carbapenemase-producing Enterobacteriales, *Pseudomonas* and *Acinetobacter* in a sample and kits for use in such methods.

### BACKGROUND ART

Carbapenemases are a group of β-lactamases that are active against various antibiotics including carbapenems. Carbapenemases belong to three classes of beta-lactamases, namely Ambler class A, Ambler class B and Ambler class D. These three classes of carbapenemases confer resistance in clinically important bacteria to carbapenems or decreased susceptibility to carbapenems. Bacteria that produce carbapenemase are therefore of interest clinically and methods for their early detection are required in order to prevent their spread and to reduce multidrug and pandrug resistance.

Two tests are widely used clinically to detect carbapenemase producing bacteria; the "Etest®" and other MIC strip products and the "Modified Hodge-Test". In the "Etest®", indicator carbapenem and carbapenem plus enzyme inhibitor antimicrobial agents against a test microorganism are provided in opposing predefined gradients on agar and used to determine the Minimum Inhibitory Concentration (MIC) of the antimicrobial agent and the inhibitory concentration ratio. In the "Modified Hodge-Test" carbapenemase producing bacteria are detected when reporter bacteria grow towards a carbapenem containing disk to produce a characteristic "clover-leaf' growth pattern around the disk. This phenomenon can be mediated by the carbapenem inactivating enzymes produced by the test isolate. Molecular detection techniques for carbapenemase genes have also been used. However, all three tests have their drawbacks. The "Etest®" and the "Modified Hodge-Test" are neither sensitive nor specific enough whilst molecular detection techniques are complicated and expensive. Moreover, all three detection methods are highly time consuming with up to 24 hours being the typical timescale for determining whether a sample contains carbapenemase producing bacteria. This is unsatisfactory when controlling nosocomial acquired infections and the transfer of drug resistance.

Recently acido-colorimetric techniques have been developed for the detection of carbapenemase producing bacteria which are reported as significantly lowering the assay time compared to "Etest®", "Modified Hodge-test" and molecular detection techniques. WO2012/175637 describes such a method in which specifically, assay times of less than 2 hours are reported. However, current rapid phenotypic tests show poor sensitivity and specificity for the detection of carbapenemase- producing *Pseudomonas* (Heinrichs *et al.,* 2015) and *Acinetobacter* in a sample. In particular, false negatives may be obtained.

Also, rapidly detecting carbapenemase producing Enterobacteriaceae is key to limiting the spread of these organisms and although there are both phenotypic and molecular methods available for detecting these, no single detection method has proven ideal for all situations (Lutgring *et* Limbago, 2016). According to the European Committee on Antimicrobial Susceptibility Testing (EUCAST, 2018), recent taxonomic studies have narrowed the definition of the family Enterobacteriaceae. Consequently, the Enterobacteriales are an order of gram-negative bacteria that includes only one family; Enterobacteriaceae. Therefore, the present invention will make reference to the order Enterobacteriales.

Consequently, there is a need to provide a method for detecting the presence of carbapenemase- producing Enterobacteriales, *Pseudomonas* and *Acinetobacter* in a sample which is not only highly specific and sensitive, but may also be easily utilized clinically.

### ADDITIONAL BACKGROUND ART

EP1557473 discloses a reagent composition that is intended to be used for detecting β-lactamase including as a β-lactamase detection substrate 3-[2,4-dinitrostyryl]-7-(2-thienylacetamido)-3-cephem-4-carboxylic acid, or 7-[2-(2-aminothiazol-4-yl)-2-(1-carboxy-1-methylethoxy-imino)acetamido]-3-(2,4-dinitrostyryl)-3-cephem-4-carboxylic acid, and at least one β-lactamase inhibitor selected from the group consisting of clavulanic acid, aztreonam, ethylenediaminetetraacetic acid, and cloxacillin, which composition can detect β-lactamases rapidly and easily with high sensitivity. The present invention also provides a detection kit including the detecting reagent composition. Further, the present invention provides a beta -lactamase detection method where a liquid specimen containing a target substance to be analyzed is brought into contact with the composition.

FR2956866 discloses a method that is intended to be used for detecting the presence of an enzyme comprising cephalosporinases, extended spectrum β-lactamases (ESBLs) and carbapenemases, comprises: (a) suspending the bacteria in a liquid composition comprising a chromogenic or fluorogenic substrate capable of releasing a chromophore or fluorophore after hydrolysis by the enzyme to detect; and (b) detecting the possible release of the chromophore or fluorophore, where the detecting the release of the chromophore or fluorophore in step (b) indicates the presence of an enzyme cephalosporinases, ESBLs and carbapenemases.

WO2009/051838 discloses methods and compositions that are intended to be used for the detection of specific β-lactamases, including class A serine carbapenemases, metallo-β-lactamases, AmpC β-lactamases, and extended-spectrum β-lactamases (ESBLs). The methods are said to include methods that are intended to permit the detection of the presence of specific β-lactamases in bacterial samples within 2-10 minutes.

WO2017/098206 discloses a method for determining whether a microorganism produces a carbapenem or penem hydrolysing p-lactamase, comprising: providing a reagent composition comprising a p-Iactam containing antibiotic, an extended-spectrum p-lactamase inhibitor and/or an AmpC inhibitor to which the carbapenem or penem hydrolysing p-lactamase is resistant, and a membrane permeabilizing agent which is ethylene glycol tetraacetic acid (EGTA) that permeabilizes the microorganism's outer membrane; contacting the microorganism suspected of producing a p-lactamase with the reagent composition; and culturing the microorganism with the reagent composition in a culture medium comprising a reporter microorganism wherein a positive test is determined by an increase in the growth of the reporter microorganism. This document also discloses an enzyme assay system and a kit for use in such a method.

EP1325923 discloses a cephem compound or pharmaceutically acceptable salt thereof that is intended to be effective for the detection or extended-spectrum β-lactamase (ESBL) producing bacteria to which third-generation cephem-related antibiotics are ineffective. More specifically, this document discloses a cephem compound or pharmaceutically acceptable salt thereof that is intended to be used for the detection of ESBL-producing bacteria.

### SUMMARY OF THE INVENTION

The inventors have found that by conducting a phenotypic test using the chromogenic cephalosporin (7R)-7-[(z)-2-(2-aminothiazol-4-yl)-(Z)-2(methoxyimino)acetamide]-3-(2,4-dinitro styryl)-3-cephem-4 carboxylic acid or a salt thereof, it is possible to detect the presence of carbapenemase producing Enterobacteriales, *Pseudomonas* and *Acinetobacter* in very short timescales.

In particular, the detection process found by the inventors does not require pre-exposure of isolates to any agents that might induce or inhibit expression prior to testing for the presence of carbapenemase, and which thus requires an additional information period.

In one aspect, the inventors have found that it is possible to detect the presence of carbapenemase producing Enterobacteriales, *Pseudomonas* and *Acinetobacter* in samples from patients suspected of being infected with such bacteria using the chromogenic cephalosporin (7R)-7-[(z)-2-(2-aminothiazol-4-yl)-(Z)-2(methoxyimino)acetamide]-3-(2,4-dinitrostyryl)-3-cephem-4 carboxylic acid or a salt thereof.

Thus, in a first aspect the present invention provides a method for detecting the presence of carbapenemase-producing Enterobacteriales, *Pseudomonas* and *Acinetobacter* in a sample from a patient, comprising: reacting a sample suspected of having carbapenemase-producing Enterobacteriales, *Pseudomonas* or *Acinetobacter* with a solution of (7R)-7-[(z)-2-(2-aminothiazol-4-yl)-(Z)-2(methoxyimino)acetamide]-3-(2,4-dinitrostyryl)-3-cephem-4 carboxylic acid or a salt thereof, and detecting a color change in the reaction medium, wherein a change in color from yellow to red indicates the presence of carbapenemase-producing Enterobacteriales, *Pseudomonas* or *Acinetobacter* in the test sample.

According to a second aspect, the present invention provides the use of (7R)-7-[(z)-2-(2-aminothiazol-4-yl)-(Z)-2(methoxyimino)acetamide]-3-(2,4-dinitrostyryl)-3-cephem-4 carboxylic acid or a salt thereof, for detecting the presence of carbapenemase-producing Enterobacteriales, *Pseudomonas* and *Acinetobacter* in a sample.

According to a third aspect the present invention also provides a kit for determining whether a microorganism produces a carbapenem-hydrolysing β-lactamase, comprising an assay disk impregnated with (7R)-7-[(z)-2-(2-aminothiazol-4-yl)-(Z)-2(methoxyimino)acetamide]-3-(2,4-dinitro styryl)-3-cephem-4 carboxylic acid or a salt thereof, and an extended spectrum β-lactam (ESBL) inhibitor, a AmpC inhibitor or a mixture thereof.

The invention also relates to a microtitre plate comprising a well or a series of wells comprising (7R)-7-[(z)-2-(2-aminothiazol-4-yl)-(Z)-2(methoxyimino)acetamide]-3-(2,4-dinitro styryl)-3-cephem-4 carboxylic acid or a salt thereof and its use in detecting the presence of carbapenemase producers in a test sample.

Also disclosed vials or microfuge tubes comprising (7R)-7-[(z)-2-(2-aminothiazol-4-yl)-(Z)-2(methoxyimino)acetamide]-3-(2,4-dinitro styryl)-3-cephem-4 carboxylic acid or a salt thereof and its use in detecting the presence of carbapenemase producers in a test sample.

### DETAILED DESCRIPTION OF THE INVENTION

Methods which allow rapid detection of carbapenemase-producing bacteria are needed in order to take necessary action to prevent the spread of antibiotic resistance and to preserve the efficacy of antibiotics such as penems and carbapenems. The method of the present invention addresses this need by providing a user with interpretable results to determine whether a sample has carbapenemase producing bacteria in a fast and reliable manner.

In particular, the method may be suitable for providing a rapid and reliable phenotypic test for detecting the presence of carbapenemase-producing Enterobacteriales, *Pseudomonas* and *Acinetobacter* spp. Accordingly, the present invention advantageously provides a method suitable for providing a rapid and reliable phenotypic test for detecting the presence of carbapenemase-producing bacteria, such as *Pseudomonas aeruginosa* which may not be rapidly and reliably tested for using other known methods and/or tests. In a first aspect, the present invention therefore provides a method for detecting the presence of carbapenemase-producing Enterobacteriales, *Pseudomonas* and *Acinetobacter* in a sample, comprising: reacting a sample suspected of having carbapenemase-producing Enterobacteriales, *Pseudomonas* or *Acinetobacter* with a solution of (7R)-7-[(z)-2-(2-aminothiazol-4-yl)-(Z)-2(methoxyimino)acetamide]-3-(2,4-dinitro styryl)-3-cephem-4 carboxylic acid or a salt thereof, and detecting a color change in the reaction medium when carbapenemase-producing Enterobacteriales, *Pseudomonas* or *Acinetobacter* are present in the test sample.

The method of the present invention may have 100% sensitivity and 100% specificity for detecting carbapenemase production in Enterobacteriales, *Pseudomonas* and *Acinetobacter* spp.

The method of the invention is based on the concept that (7R)-7-[(z)-2-(2-aminothiazol-4-yl)-(Z)-2(methoxyimino)acetamide]-3-(2,4-dinitrostyryl)-3-cephem-4 carboxylic acid or a salt thereof may be hydrolysed by a β-lactamse such as a carbapenemase. The hydrolysis of ([(7R)-7-[(z)-2-(2-aminothiazol-4-yl)-(Z)-2(methoxyimino)acetamide]-3-(2,4-dinitrostyryl)-3-cephem-4 carboxylic acid, results in an observable colour change from yellow to red.

Preferably, the method of the present invention is conducted on samples obtained from a subject which is suspected of being infected with carbapenemase producing bacteria. The sample may be any biological sample obtained from a subject such as fluids, tissues or cell samples. Preferably the sample may be from isolated cell culture or is a urine sample. The sample may be obtained by known methods from the subject which may be a mammal. Preferably the subject from which the sample is obtained is a human.

The method of the present invention may be used to detect any Enterobacteriales,*Pseudomonas* or *Acinetobacter* carbapenemase-producing bacteria. Preferably, the carbapenemase-producing bacteria are of clinical importance such as bacteria that are responsible for nosocomial or community-acquired infections.

Typically, the concentration of (7R)-7-[(z)-2-(2-aminothiazol-4-yl)-(Z)-2(methoxyimino)acetamide]-3-(2,4-dinitrostyryl)-3-cephem-4 carboxylic acid or a salt thereof, substrate used in the method of the invention is from 0.1 mg/ml to 10 mg/ml, more preferably from 1 mg/ml to 5 mg/ml and even more preferably from 2 mg/ml to 3 mg/ml.

According to some embodiments, the carbapenemase-producing bacteria are lysed prior to reaction with (7R)-7-[(z)-2-(2-aminothiazol-4-yl)-(Z)-2(methoxyimino)acetamide]-3-(2,4-dinitrostyryl)-3-cephem-4 carboxylic acid or a salt thereof. Lysis of the bacteria may be performed by any known technique. Optionally, an amount of Enterobacteriales, *Pseudomonas* or *Acinetobacter* spp, may be resuspended in a protein extraction buffer/ reagent in a microfuge tube or vial prior to mixing. Mixing may be performed by vortexing for a suitable period to ensure thorough mixing. Preferably, mixing may be achieved by vortexing the sample for a period from about 2 to about 10 seconds, e.g. for 5 seconds. The tube may then be incubated for a period of between 5 and 30 minutes at a suitable temperature. Optionally, the tube may be incubated for a period of about 10 minutes. Suitable temperatures may be from about 25 °C to about 45 °C. Preferably the tube may be incubated from about 35 °C to about 37 °C.

In some embodiments, sample lysis is facilitated by the combination of a polymyxin, glycopeptide and polypeptide antibiotic.

The reaction of the sample, which may be a lysed sample, with (7R)-7-[(z)-2-(2-aminothiazol-4-yl)-(Z)-2(methoxyimino)acetamide]-3-(2,4-dinitrostyryl)-3-cephem-4 carboxylic acid or a salt thereof, may be carried out over a period of time sufficient to observe a color change. Preferably the colour change should be observed in less than 1 hour. More preferably the colour change maybe observed in less than 30 minutes. Most preferably a colour change may be observed in between about 5 and about 20 minutes.

The reaction of the sample with (7R)-7-[(z)-2-(2-aminothiazol-4-yl)-(Z)-2(methoxyimino)acetamide]-3-(2,4-dinitrostyryl)-3-cephem-4 carboxylic acid or a salt thereof may be carried out at any suitable temperature. Preferably, the reaction may be carried out from about 5 °C to about 40 °C. For example, the reaction may be carried out from about 15 °C to about 30 °C, such as at room temperature. Preferably, the reaction may be carried out from about 20 °C to about 37 °C, and most preferably at 35 °C.

The chromogenic cephalosporin (7R)-7-[(z)-2-(2-aminothiazol-4-yl)-(Z)-2(methoxyimino)acetamide]-3-(2,4-dinitrostyryl)-3-cephem-4 carboxylic acid or a salt thereof is also susceptible to hydrolysis by extended spectrum beta-lactamases (ESBLs) and AmpCs. However, the effect of ESBL and AmpC enzymes may be eliminated by the addition of suitable inhibitor compounds. Accordingly, when such inhibitors are present, a change in colour indicates the presence of a carbapenemase.

Accordingly, in one embodiment the method further comprises an extended spectrum β-lactam (ESBL) inhibitor, an AmpC inhibitor or a mixture thereof. Any suitable inhibitor may be used. Some suitable inhibitors include clavulanic acid, cloxacillin and mixtures thereof. Optionally, the inhibitor comprises at least one of clavulanic acid at a concentration of between about 30 µg/ml to about 50 µg/ml and cloxacillin at a concentration of between about 300 µg/ml to about 500 µg/ml. Where in inhibitor comprises clavulanic acid and cloxacillin, these may be mixed together in equal parts to yield the inhibitor mix. Alternatively, different amounts and/or additional components may be used. Also, any carbapenem or penem could be used alone or in combinations, with both ESBL and AmpC inhibitors to further eliminate ESBL and AmpC activity.

The selection of inhibitors that inhibit extended spectrum beta-lactamases (ESBLs) and AmpCs allows for the visualisation of the whole spectrum of carbapenemase expression.

In one embodiment of the present invention, the (7R)-7-[(z)-2-(2-aminothiazol-4-yl)-(Z)-2(methoxyimino)acetamide]-3-(2,4-dinitrostyryl)-3-cephem-4 carboxylic acid or a salt thereof may be dissolved in a solution comprising at least one of an organic solvent, an organic acid and zinc sulphate. Optionally, the organic solvent may be a polar, aprotic solvent. A suitable solvent may be DMSO. The organic acid may be any suitable organic acid. Suitable organic acids may include those from the linear saturated dicarboxylic acid groupsuch as oxalic acid, malonic acid, succinic acid, glutaric acid, adipic acid, pimelic acid, suberic acid, azelaic acid or sebacic acid.

It shall be understood that the reaction of the sample may be carried out utilizing a sequential sequence steps for example, including at least some of mixing an inhibitor mix, dissolving (7R)-7-[(z)-2-(2-aminothiazol-4-yl)-(Z)-2(methoxyimino)acetamide]-3-(2,4-dinitrostyryl)-3-cephem-4 carboxylic acid or a salt thereof in a solution comprising at least one of an organic solvent, an organic acid and zinc sulphate, suspending an amount of carbapenemase-producing Enterobacteriales, *Pseudomonas* and *Acinetobacter* in an amount of protein extractor reagent, for texting and or heating the suspended carbapenemase-producing Enterobacteriales, *Pseudomonas* and *Acinetobacter* to produce a live sample, and adding the lysed to sample to the (7R)-7-[(z)-2-(2-aminothiazol-4-yl)-(Z)-2(methoxyimino)acetamide]-3-(2,4-dinitrostyryl)-3-cephem-4 carboxylic acid (or a salt thereof) solution with an amount of the inhibitor mix.

In other embodiments, the reaction process may be carried out via a non-sequential reaction process, where three or more or of (7R)-7-[(z)-2-(2-aminothiazol-4-yl)-(Z)-2(methoxyimino)acetamide]-3-(2,4-dinitrostyryl)-3-cephem-4 carboxylic acid or a salt thereof, an organic solvent, an organic acid, zinc sulphate are added to a sample of carbapenemase-producing Enterobacteriales, *Pseudomonas* and *Acinetobacter* and lysed in situ simultaneously with the reaction process.

For example, the reaction of the sample may be carried out utilizing a "one-pot" synthesis technique in which an un-lysed sample may be reacted with (7R)-7-[(z)-2-(2-aminothiazol-4-yl)-(Z)-2(methoxyimino)acetamide]-3-(2,4-dinitrostyryl)-3-cephem-4 carboxylic acid or a salt thereof, an organic solvent and an organic salt. Optionally, the organic solvent may be DMSO and the organic acid may be a linear saturated dicarboxylic acid, such as succinic acid.

In a second aspect, the present invention provides the use of (7R)-7-[(z)-2-(2-aminothiazol-4-yl)-(Z)-2(methoxyimino)acetamide]-3-(2,4-dinitrostyryl)-3-cephem-4 carboxylic acid or a salt thereof for detecting the presence of carbapenemase-producing Enterobacteriales, *Pseudomonas* or *Acinetobacter* in a sample.

The (7R)-7-[(z)-2-(2-aminothiazol-4-yl)-(Z)-2(methoxyimino)acetamide]-3-(2,4-dinitrostyryl)-3-cephem-4 carboxylic acid or a salt thereof may optionally be in powder form. The ([(7R)-7-[(z)-2-(2-aminothiazol-4-yl)-(Z)-2(methoxyimino)acetamide]-3-(2,4-dinitrostyryl)-3-cephem-4 carboxylic acid or a salt thereof may be dissolved in a mixture of DMSO to and succinic acid, followed by the addition of zinc sulphate. For Example, the (7R)-7-[(z)-2-(2-aminothiazol-4-yl)-(Z)-2(methoxyimino)acetamide]-3-(2,4-dinitrostyryl)-3-cephem-4 carboxylic acid or a salt thereof may be dissolved in 1 part DMSO to from 3 to 20 parts succinic acid, followed by the addition of zinc sulphate to give a final concentration of from 0.1mM to 10mM. Preferably, the (7R)-7-[(z)-2-(2-aminothiazol-4-yl)-(Z)-2(methoxyimino)acetamide]-3-(2,4-dinitrostyryl)-3-cephem-4 carboxylic acid or a salt thereof may be dissolved in of 1 part DMSO to 5 to 9 parts succinic acid. Optionally, the (7R)-7-[(z)-2-(2-aminothiazol-4-yl)-(Z)-2(methoxyimino)acetamide]-3-(2,4-dinitrostyryl)-3-cephem-4 carboxylic acid or a salt thereof may be dissolved in a 1 part DMSO to 7 parts succinic acid, followed by the addition of zinc sulphate to give a final concentration of 1mM.

An amount of lysed or whole cell sample may be added in approximately equal parts to the (7R)-7-[(z)-2-(2-aminothiazol-4-yl)-(Z)-2(methoxyimino)acetamide]-3-(2,4-dinitrostyryl)-3-cephem-4 carboxylic acid or a salt thereof solution. The lysed or whole cell sample may be added to the (7R)-7-[(z)-2-(2-aminothiazol-4-yl)-(Z)-2(methoxyimino)acetamide]-3-(2,4-dinitrostyryl)-3-cephem-4 carboxylic acid or a salt thereof solution at room temperature. Optionally, about 20µl to about 100µl of the lysed sample and (7R)-7-[(z)-2-(2-aminothiazol-4-yl)-(Z)-2(methoxyimino)acetamide]-3-(2,4-dinitrostyryl)-3-cephem-4 carboxylic acid or a salt thereof solution may be used.

An amount of inhibitor may also be added. Any suitable inhibitor or mixtures thereof may be used in amounts from 20µl to about 200µl. Preferably, about 50µl to about 150µl of inhibitor may be used. Optionally about 100µl of an inhibitor comprising clavulanic acid and cloxacillin in equal parts made up to a final concentration of about 40µg/ml and 400 µg/ml may be added.

Alternatively, a different inhibitor or mixture of inhibitors, or ratios thereof may be added at an equivalent final concentration.

The contents of the resultant solution may be monitored for a period of up to an hour, and preferably for up to 30 minutes, to determine if a colour change occurs. Preferably the solution may only need to be monitored for a period of between 5 and 30 minutes.

The occurrence of a colour change from yellow to orange/red may indicate the presence of carbapenemase-producing Enterobacteriales, *Pseudomonas* or *Acinetobacter* in the test sample.

In order to test whether a carbapenemase-producing Enterobacteriales, *Pseudomonas* or *Acinetobacter* is present in a test sample, an amount of Enterobacteriales, *Pseudomonas* or *Acinetobacter* spp may be suspended in a protein extraction buffer/reagent. The solution may then be mixed and incubated to produce a lysed sample. A portion of the lysed sample may be added to an amount of (7R)-7-[(z)-2-(2-aminothiazol-4-yl)-(Z)-2(methoxyimino)acetamide]-3-(2,4-dinitrostyryl)-3-cephem-4 carboxylic acid or a salt thereof and optionally an amount of the inhibitor mix, with a positive test verified by the observance of a colour change.

The amount of Enterobacteriales, *Pseudomonas* or *Acinetobacter* spp used may be from about 0.1µl to about 10µl. For example, about 0.2µl, 0.5µl, 1µl, 2µl or 5µl may be used. The amount of protein extraction reagent used may be between 50 and 250µl. For example, about 50µl, 75µl, 100µl, 150µl may be used. Preferably about 1µl of Enterobacteriales, Pseudomonas or Acinetobacter spp and about 100µl of protein extraction buffer/reagent may be used. Any suitable protein extraction buffer/reagent may be used.

Mixing may be achieved using any suitable technique, including, but not limited to stirring, shaking and vortex mixing. For example, the solution may be vortex mixed for a period from about 1 to 60 seconds, such as for about 5 seconds. Mixing may occur prior to, and/or during, incubation to produce a lysed sample. Incubation may be performed at any suitable temperature and for any suitable length of time. For example, incubation may be performed at a temperature from about 30 to 45 °C, preferably about 35 to 37 °C, for about 1 to about 30 minutes, preferably about 10 minutes.

Optionally, the test may be performed on an un-lysed sample.

Any suitable portion of the lysed (or un-lysed) sample may be added to any suitable amount of the (7R)-7-[(z)-2-(2-aminothiazol-4-yl)-(Z)-2(methoxyimino)acetamide]-3-(2,4-dinitrostyryl)-3-cephem-4 carboxylic acid or a salt thereof solution, optionally with an amount of the inhibitor mix. Optionally, about 10µl to about 100µl, for example about 50µl, of a lysed (or un-lysed) sample may be added to about 20µ to about 100µl, for example about 50µl of the (7R)-7-[(z)-2-(2-aminothiazol-4-yl)-(Z)-2(methoxyimino)acetamide]-3-(2,4-dinitrostyryl)-3-cephem-4 carboxylic acid or a salt thereof solution, optionally in addition to about 20 to 200µl, for example about 100µl, of the inhibitor mix.

Preferably, the sample, (7R)-7-[(z)-2-(2-aminothiazol-4-yl)-(Z)-2(methoxyimino)acetamide]-3-(2,4-dinitrostyryl)-3-cephem-4 carboxylic acid or a salt thereof solution and optionally, inhibitor mix, may be added at room temperature.

In a third aspect, the present invention provides a kit for determining whether a microorganism produces a carbapenem-hydrolysing β-lactamase, may comprise an assay disk impregnated with ([(7R)-7-[(z)-2-(2-aminothiazol-4-yl)-(Z)-2(methoxyimino)acetamide]-3-(2,4-dinitro styryl)-3-cephem-4 carboxylic acid) or a salt thereof, and an extended spectrum β-lactam (ESBL) inhibitor, a AmpC inhibitor or a mixture thereof.

In a further aspect the present disclosure provides a microfluidic device or electrochemical sensing device for detecting the presence of a carbapenemase producing Enterobacteriales, *Pseudomonas* or *Acinetobacter* wherein the device comprises a substrate which is (7R)-7-[(z)-2-(2-aminothiazol-4-yl)-(Z)-2(methoxyimino)acetamide]-3-(2,4-dinitro styryl)-3-cephem-4 carboxylic acid or a salt thereof.

The microfluidic device may be any device which gives results that may be read phenotypically. For example, the microfluidic device could be a micro-capillary film.

The electrochemical sensing device could be any device that will give a signal following the hyrolysis of (7R)-7-[(z)-2-(2-aminothiazol-4-yl)-(Z)-2(methoxyimino)acetamide]-3-(2,4-dinitro styryl)-3-cephem-4 carboxylic acid or a salt thereof. For example, the electrochemical sensing device could be an electrode, chip, kit, or ironically charged membrane.

The reaction of a lysed sample, with a reagent kit is conducted over a period of time sufficient to observe a color change in the (7R)-7-[(z)-2-(2-aminothiazol-4-yl)-(Z)-2(methoxyimino)acetamide]-3-(2,4-dinitrostyryl)-3-cephem-4 carboxylic acid or a salt thereof solution when carbapenemase-producing Enterobacteriales, *Pseudomonas* or *Acinetobacter* are present in the test sample. Typically, the colour change is observed less than 1 hour and more preferably less than 30 minutes from mixing the sample with the buffer/reagent. Typically, the reaction of the sample with a reagent kit is carried out at a temperature comprised from 5°C to 40°C, preferably from 15°C to 30°C, and more preferably at room temperature.

The reagent kit may be used to provide a very rapid way of identifying an infection with carbapenemase-producing Enterobacteriaceae, *Pseudomonas* or *Acinetobacter* bacteria with high reliability and accuracy, according to the method of the present invention.

### Figures

Figure 1 shows phenotypic results for *Pseudomonas* spp.
Figure 2 shows phenotypic results for *Acinetobacter baumanii.*
Figure 3 shows comparative testing of *Pseudomonas* and *Acinetobacter* spp. using a method of the present invention and the standard technique.

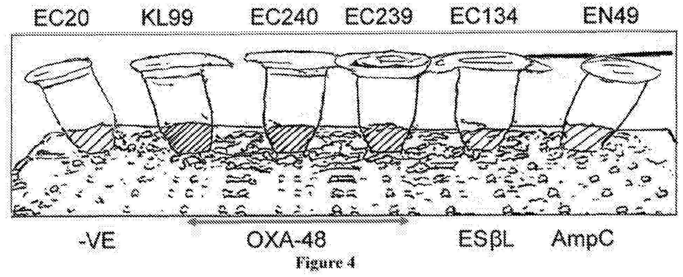

Figure 4 shows phenotypic results for the detection of carbapenemase producing Enterobacteriales

The invention will further be illustrated in view of the following and examples.

### Example 1

A test solution comprising an inhibitor mix and ([(7R)-7-[(z)-2-(2-aminothiazol-4-yl)-(Z)-2(methoxyimino)acetamide]-3-(2,4-dinitrostyryl)-3-cephem-4 carboxylic acid, was produced and used to determine the presence of carbapenemase-producing *Pseudomonas* or *Acinetobacter* in a number of tests samples, as set out in Tables 1 and 2.

The test solution was made up as follows:
1) Clavulanic acid and cloxacillin were made up to a final concentration of 40µg/ml and 400µg/ml and mixed together in equal parts to yield the inhibitor mix.
2) (7R)-7-[(z)-2-(2-aminothiazol-4-yl)-(Z)-2(methoxyimino)acetamide]-3-(2,4-dinitrostyryl)-3-cephem-4 carboxylic acid or a salt thereof in powder form was dissolved in 1 part DMSO to 7 parts dicarboxylic acid, followed by the addition of zinc sulphate to a final concentration of 1mM.
3) A 1µl loopful of either *Pseudomonas* or *Acinetobacter* spp was suspended in 100µl of a commercially available protein extraction buffer/reagent or 100µl of a extraction buffer comprising of 50mM Tris HCl, 100mM NaCl, and 1% Triton X100 in a microfuge tube.
4) The suspended *Pseudomonas* or *Acinetobacter* spp was mixed by vortexing for a period of 5 seconds prior to heating of the sample in the microfuge to 35°C-37°C and incubating for a period of 10 minutes to produce a lysed sample.
5) 50µl of the lysed sample was added to 50µl of the (7R)-7-[(z)-2-(2-aminothiazol-4-yl)-(Z)-2(methoxyimino)acetamide]-3-(2,4-dinitrostyryl)-3-cephem-4 carboxylic acid or a salt thereof solution and 100µl of the inhibitor mix in a well of a 96-microtitre plate at room temperature.
6) The contents of the well were mixed by pipetting during the addition of the cell lysate.
7) The sample was visually monitored for up to 30 minutes, with the solution colour noted at 5, 10, 20 and 30 minutes from mixing of the sample with the ([(7R)-7-[(z)-2-(2-aminothiazol-4-yl)-(Z)-2(methoxyimino)acetamide]-3-(2,4-dinitrostyryl)-3-cephem-4 carboxylic acid or a salt thereof solution.

**Table 1: Panel of Pseudomonas spp and result obtained with candidate chromogenic substrate**

| Organi sm | Carbapenemase Resistance | A TCC/NCTC | 10-100µl of inhibitors | | | |
|---|---|---|---|---|---|---|
| | | | Result: Red/Orange = +(pos) Yellow = -(neg) | | | |
| | | | Time after addition of the substrate, CX & CLV (minutes) | | | |
| | | | 5 | 10 | 20 | 30 |
| *Pseudomonas aeruginosa* | No | - | - | - | - | - |
| *Pseudomonas aeruginosa* | No | - | - | - | - | - |
| *Pseudomonas aeruginosa* | No | ATCC 27853 | - | - | - | - |
| | | NCTC 12903 | | | | |
| *Pseudomonas aeruginosa* | No | - | - | - | - | - |
| *Pseudomonas aeruginosa* | No | ATCC 25668 | - | - | - | - |
| | | NCT 10662 | | | | |
| *Pseudomonas aeruginosa* | No | - | - | - | - | - |
| *Pseudomonas* spp. | No | - | - | - | - | - |
| *Pseudomonas* spp. | No | - | - | - | - | - |
| *Pseudomonas* spp. | No | - | - | - | - | - |
| *Pseudomonas* spp. | No | - | - | - | - | - |
| *Pseudomonas* spp. | NDM-1 | - | + | + | + | + |
| *Pseudomonas* spp. | NDM-1 | - | + | + | + | + |
| *Pseudomonas aeruginosa* | No | - | - | - | - | - |
| *Pseudomonas aeruginosa* | NDM-1 | - | + | + | + | + |
| *Pseudomonas aeruginosa* | No | - | - | - | - | - |
| *Pseudomonas aeruginosa* | No | - | - | - | - | - |
| *Pseudomonas* spp. | IMP-1 | - | + | + | + | + |
| *Pseudomonas aeruginosa* | No | - | - | - | - | - |
| *Pseudomonas* spp. | NDM-1 | - | + | + | + | + |
| *Pseudomonas aeruginosa* | VIM-4 | - | + | + | + | + |
| *Pseudomonas aeruginosa* | VIM-2 | - | + | + | + | + |
| *Pseudomonas aeruginosa* | IMP-1 | - | + | + | + | + |
| *Pseudomonas aeruginosa* | No | - | - | - | - | - |
| *Pseudomonas aeruginosa* | No | - | - | - | - | - |
| *Pseudomonas aeruginosa* | VIN-10 | NCTC 13437 | + | + | + | + |

As shown in Table 1, (7R)-7-[(Z)-2-(2-Aminothiazol-4-yl) - (Z)-2(methoxyimino)acetamide]-3-(2,4-dinitrostyryl)-3-cephem-4 carboxylic acid or a salt thereof successfully detected all 9 carbapenemase producing *Pseudomonas* spp. The sensitivity of the method of the present invention was 100 %, as was the specificity.

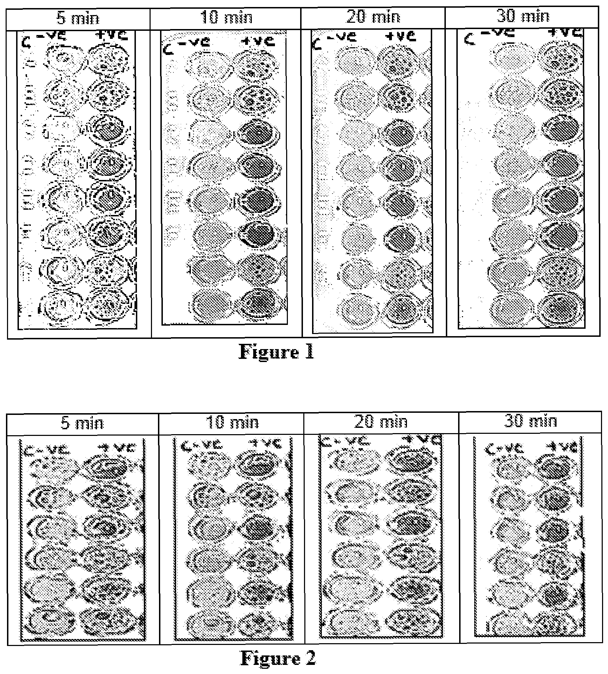

Corresponding phenotypic results for *Pseudomonas* spp. are depicted in Figure 1.

These tests show that the method of the present invention is very sensitive for the detection of NDM-1, IMP and VIMs in *Pseudomonas* spp. Furthermore the yellow colour of the (7R)-7-[(z)-2-(2-aminothiazol-4-yl)-(Z)-2(methoxyimino)acetamide]-3-(2,4-dinitrostyryl)-3-cephem-4 carboxylic acid or a salt thereof solution is shown to undergo a significant change to a red colour within 5 minutes after the addition of lysed bacterial samples at room temperature. While a stronger red may develop over longer times, the colour change after 5 minutes is enough to show a positive reaction.

**Table 2: Panel of Acinetobacter spp and result obtained with candidate chromogenic substrate**

| Organi sm | Carbapenemase producer | A TCC/NCTC | 10-100µl of inhibitors | | | |
|---|---|---|---|---|---|---|
| | | | Result: Red/Orange = +(pos) Yellow = -(neg) | | | |
| | | | Time after addition of the substrate, CX & CLV (minutes) | | | |
| | | | 5 | 10 | 20 | 30 |
| *Acinetobacter* spp. | No | - | - | - | - | - |
| *Acinetobacter* spp. | No | ACTC 15309 | - | - | - | - |
| | | NCTC 5866 | | | | |
| *Acinetobacter* spp. | No | - | - | - | - | - |
| *Acinetobacter baumanii* | No | - | - | - | - | - |
| *Acinetobacter* spp. | No | - | - | - | - | - |
| *Acinetobacter* spp. | No | - | - | - | - | - |
| *Acinetobacter* spp. | No | - | - | - | - | - |
| *Acinetobacter* spp. | No | - | - | - | - | - |
| *Acinetobacter haemolyicus* | No | - | - | - | - | - |
| *Acinetobacter Iwoffii* | No | - | - | - | - | - |
| *Acinetobacter baumanii* | No | - | - | - | - | - |
| *Acinetobacter baumanii* | No | - | - | - | - | - |
| *Acinetobacter baumanii* | MBL | - | + | + | + | + |
| *Acinetobacter* spp. | No | - | - | - | - | - |
| *Acinetobacter* spp. | No | - | - | - | - | - |
| *Acinetobacter* spp. | No | - | - | - | - | - |
| *Acinetobacter* spp. | No | - | - | - | - | - |
| *Acinetobacter baumanii* | OXA-23 | NCTC 13301 | + | + | + | + |
| *Acinetobacter baumanii* | OXA-25 | NCTC 13302 | + | + | + | + |
| *Acinetobacter baumanii* | OXA-26 | NCTC 13303 | + | + | + | + |
| *Acinetobacter baumanii* | OXA-27 | NCTC 13304 | + | + | + | + |
| *Acinetobacter baumanii* | OXA-58 | NCTC 13305 | + | + | + | + |

As shown in Table 2, (7R)-7-[(Z)-2-(2-Aminothiazol-4-yl) - (Z)-2(methoxyimino)acetamide]-3-(2,4-dinitrostyryl)-3-cephem-4 carboxylic acid or a salt thereof successfully detected all 5 carbapenemase producing *Acinetobacter baumanii.* The sensitivity of the method of the present invention was 100 %, as was the specificity.

Corresponding phenotypic results for *Acinetobacter baumanii* are depicted in Figure 2.

These tests show that the method of the present invention is very sensitive for the detection of OXAs in *Acinetobacter* spp. Furthermore the yellow colour of the (7R)-7-[(z)-2-(2-aminothiazol-4-yl)-(Z)-2(methoxyimino)acetamide]-3-(2,4-dinitrostyryl)-3-cephem-4 carboxylic acid or a salt thereof solution is shown to undergo a significant change to a red colour within 5 minutes after the addition of lysed bacterial samples at room temperature. While a stronger red may develop over longer times, the colour change after 5 minutes is enough to show a positive reaction.

### Example 2

A test solution was made up according to the method of Example 1 and was used to test five *Pseudomonas* isolates; of which 4 isolates were resistant to the carbapenems and 1 was a carbapenemase non-producer. Of the 4 carbapenemase producing *Pseudomonas* isolates, 2 were NDM-1s, whilst the remaining 2 were VIMs. Six *Acinetobacter* isolates were also tested; of which 5 isolates were resistant to the carbapenems and 1 was a carbapenemase non-producer. All the 5 carbapenemase producing *Acinetobacter* isolates were OXAs.

Corresponding tests for each *Pseudomonas* and *Acinetobacter* isolate were also tested using the commercially available colorimetric test for detecting strains with reduced susceptibility to the carbapenems, known to the skilled person in the field. Following standard instruction, the comparative tests colorimetric based testing technique were performed at 35°C - 37°C, while the tests carried out according to a method of the present invention were performed at room temperature.

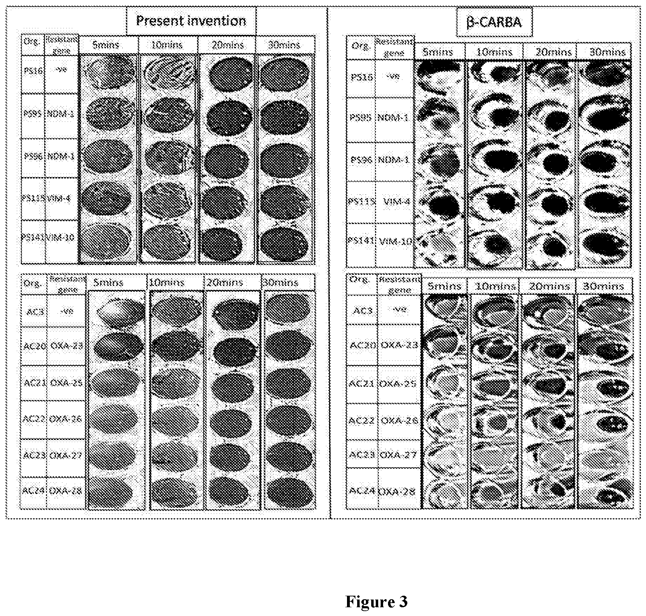

The results of these test are depicted in Table 3 with the observed colour changes shown in Figure 3.

The test included a negative control *Pseudomonas* and *Acinetobacter* isolate consisting of a carbapenemase negative *Pseudomonas aeruginosa* and an *Acinetobacter iwoffi* isolate. All of the remaining isolates were carbapenemase producers.

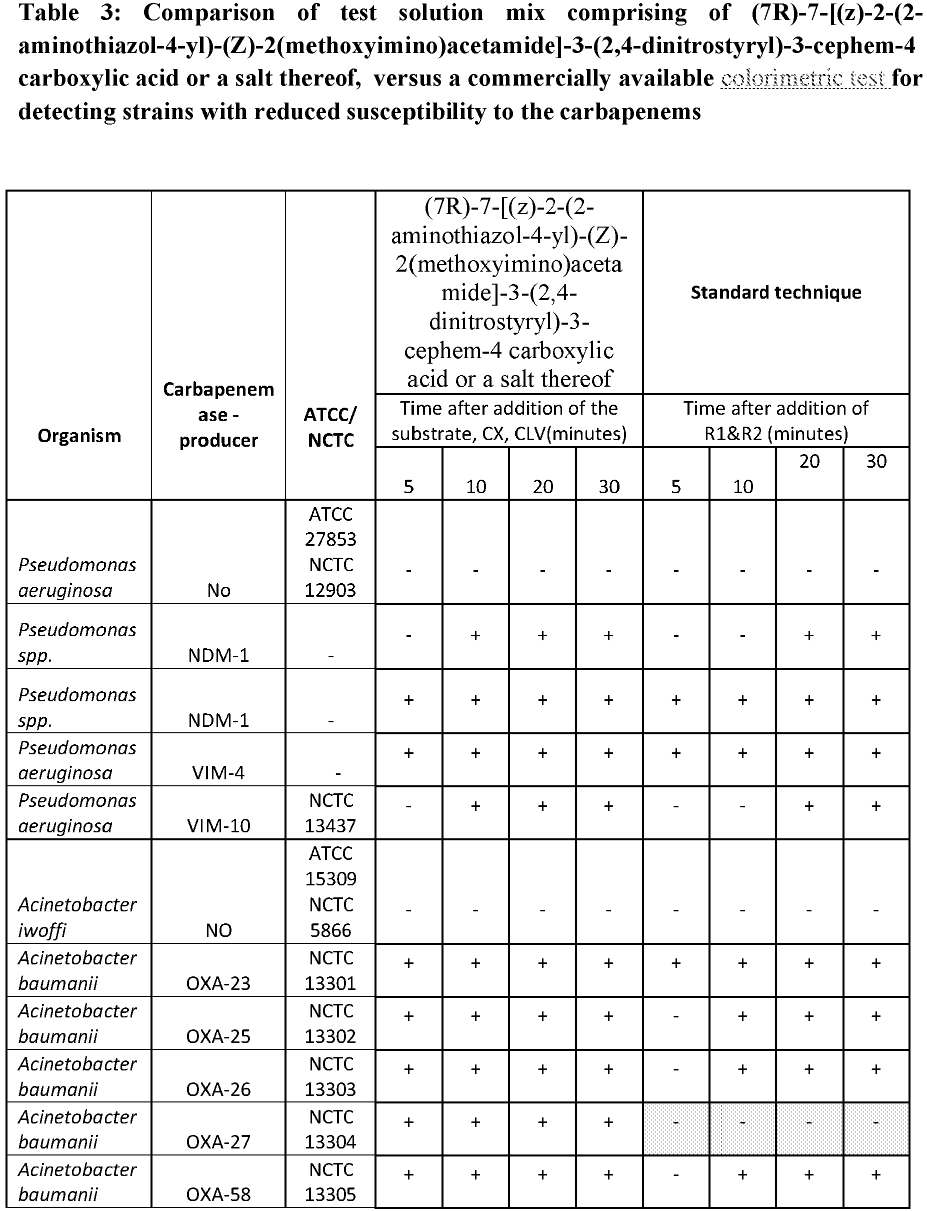

As shown in Table 3, of the 11 organisms tested during the comparison of (7R)-7-[(z)-2-(2-aminothiazol-4-yl)-(Z)-2(methoxyimino)acetamide]-3-(2,4-dinitrostyryl)-3-cephem-4 carboxylic acid or a salt thereof and a commercially available pH dependent diagnostic method, (7R)-7-[(z)-2-(2-aminothiazol-4-yl)-(Z)-2(methoxyimino)acetamide]-3-(2,4-dinitrostyryl)-3-cephem-4 carboxylic acid or a salt thereof, successfully detected all nine of the carbapenemase producing *Pseudomonas* and *Acinetobacter* isolates, while tests using the standard technique did not detect carbapenemase production in one *Acinetobacter* isolate (highlighted in table). The false negative that was misidentified using the standard test, was an OXA-27.

Accordingly, it is shown that the standard technique has a specificity of 100%, but only an 89% sensitivity, while the method according to the claimed invention has a sensitivity and a specificity of 100 %. The method of the present invention therefore provides a rapid method for detecting the presence of carbapenemase-producing *Pseudomonas* or *Acinetobacter* in the test sample, with an improved sensitivity compared to that of the known techniques.

### Example 3.

Two test solutions were made up according to the method of Example 1. One of the solutions was exactly as described in Example 1 and the other solution utilized the chromogenic cephalosporin HMRZ-86 in place of (7R)-7-[(z)-2-(2-aminothiazol-4-yl)-(Z)-2(methoxyimino)acetamide]-3-(2,4-dinitrostyryl)-3-cephem-4 carboxylic acid or a salt thereof.

Both test solutions were then used to test 68 *Pseudomonas* isolates; of which 8 isolates were resistant to the carbapenems and 60 were carbapenemase non-producers. Of the 8 carbapenemase producing *Pseudomonas* isolates, 4 were NDM-1s, 2 were VIMs, while the remaining 2 were IMPs.

Both test solutions either using HMRZ-86 or (7R)-7-[(z)-2-(2-aminothiazol-4-yl)-(Z)-2(methoxyimino)acetamide]-3-(2,4-dinitrostyryl)-3-cephem-4 carboxylic acid, were carried out according to a method of the present invention (performed at room temperature). The results of the tests are depicted in Table 4

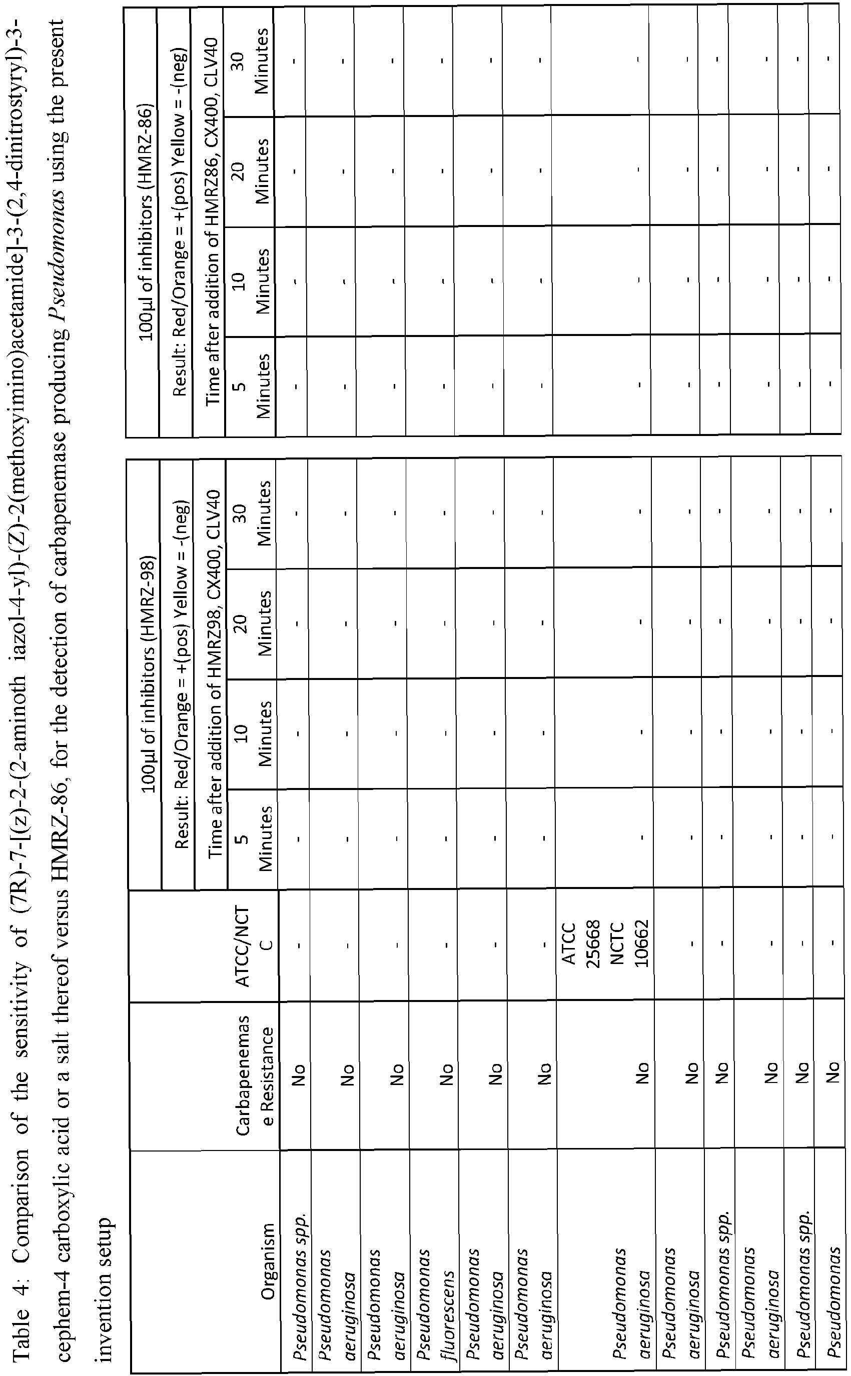

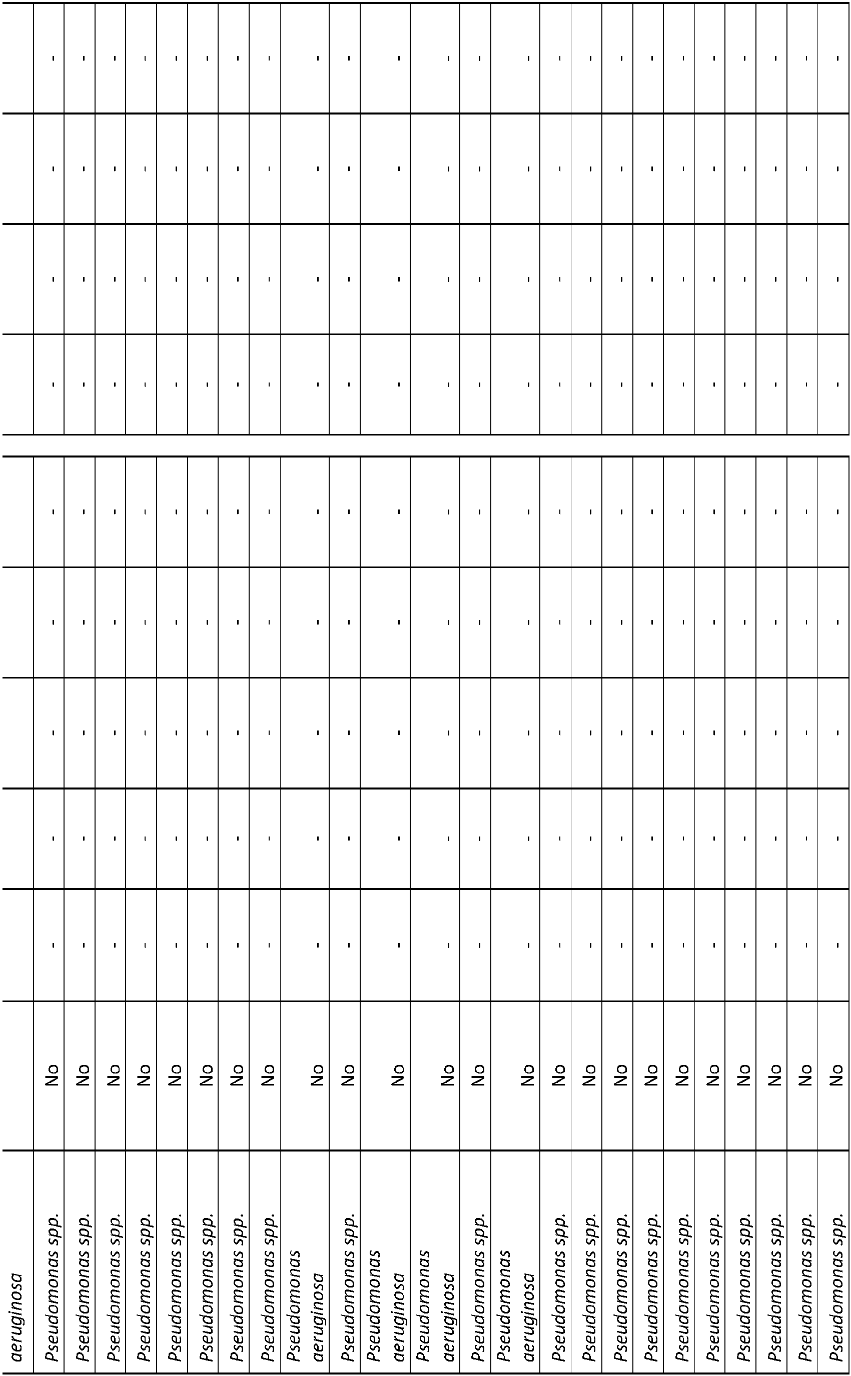

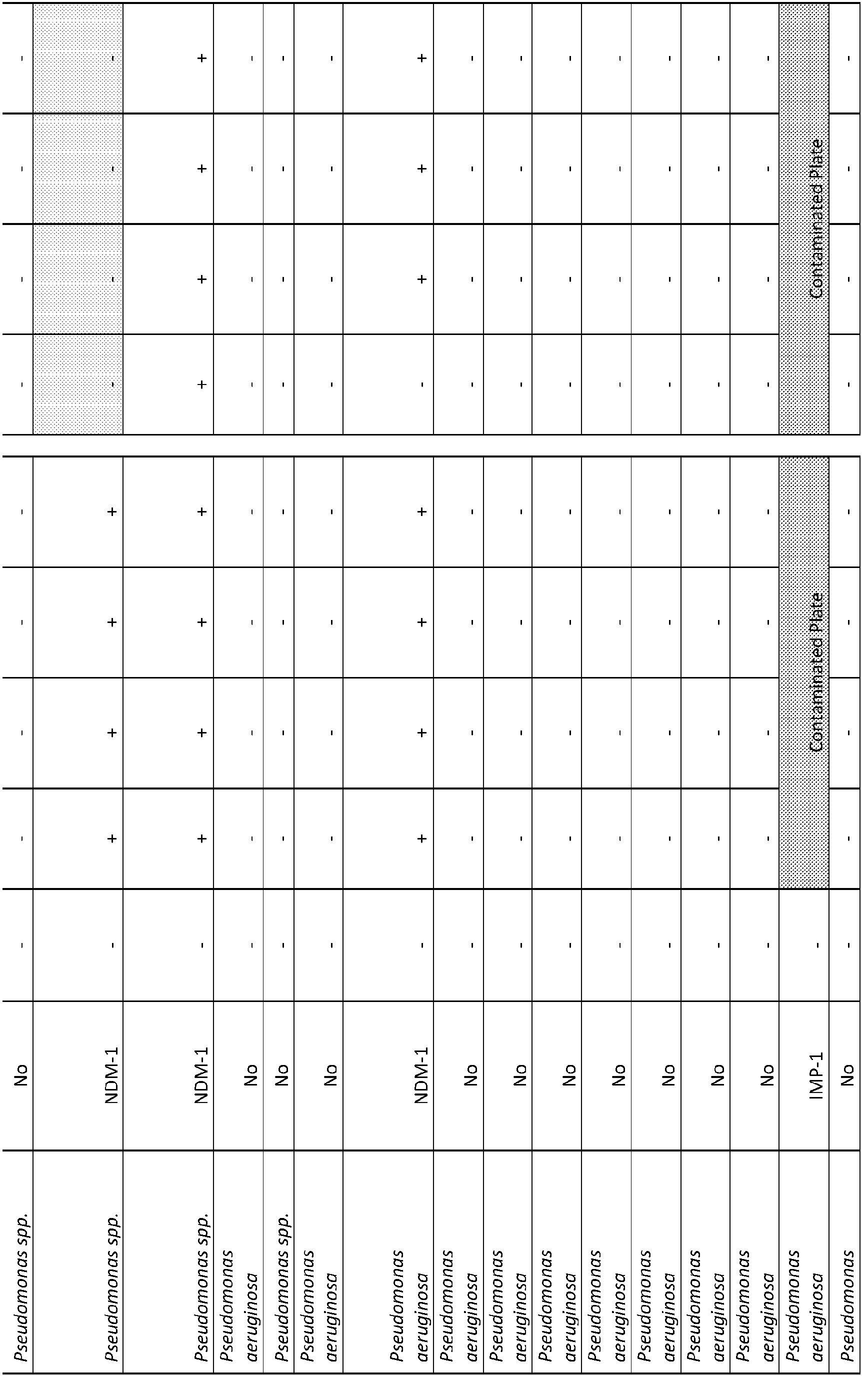

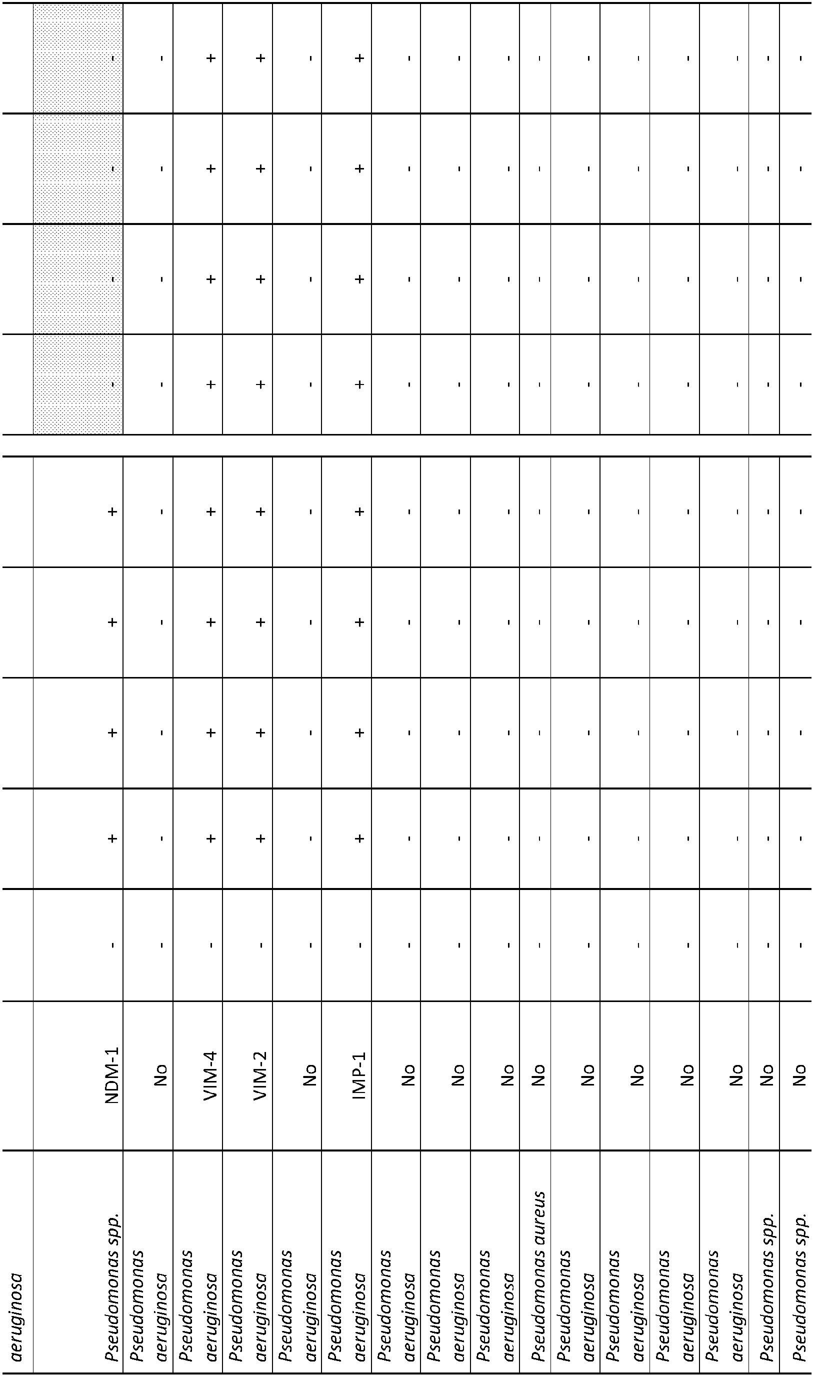

As shown in Table 4, of the 8 carbapenemase producing Pseudomonas organisms tested during the comparison of (7R)-7-[(z)-2-(2-aminothiazol-4-yl)-(Z)-2(methoxyimino)acetamide]-3-(2,4-dinitrostyryl)-3-cephem-4 carboxylic acid, versus HMRZ, (7R)-7-[(z)-2-(2-aminothiazol-4-yl)-(Z)-2(methoxyimino)acetamide]-3-(2,4-dinitrostyryl)-3-cephem-4 carboxylic acid, successfully detected all eight of the carbapenemase producing *Pseudomonas* isolates, while tests using the HMRZ-86 in place of (7R)-7-[(z)-2-(2-aminothiazol-4-yl)-(Z)-2(methoxyimino)acetamide]-3-(2,4-dinitrostyryl)-3-cephem-4 carboxylic acid did not detect two NDM-1 producing isolates (highlighted in table 4).

Accordingly, it is shown that the present invention technique has a sensitivity and specificity of 100%, whilst the candidate chromogenic substrate with HMRZ-86 results in a 75% sensitivity. The method of the present invention with (7R)-7-[(z)-2-(2-aminothiazol-4-yl)-(Z)-2(methoxyimino)acetamide]-3-(2,4-dinitrostyryl)-3-cephem-4 carboxylic acid therefore boasts higher sensitivity for the rapid detection of carbapenemase-producing *Pseudomonas* or *Acinetobacter* in test samples,

### Example 4

A dicarboxylic acid buffered test solution comprising ([(7R)-7-[(z)-2-(2-aminothiazol-4-yl)-(Z)-2(methoxyimino)acetamide]-3-(2,4-dinitrostyryl)-3-cephem-4 carboxylic acid, an inhibitor mix, zinc sulphate and a bulking agent, was produced and used to determine the presence of carbapenemase-producing Enterobacteriales, *Pseudomonas* or *Acinetobacter* in 147 test isolates, as set out in Table 5.

The test solution was made up as follows:
1) (7R)-7-[(z)-2-(2-aminothiazol-4-yl)-(Z)-2(methoxyimino)acetamide]-3-(2,4-dinitrostyryl)-3-cephem-4 carboxylic acid, in powder form was dissolved in 1 part DMSO to 7 parts dicarboxylic acid,
2) The solution from step 1 above was then buffered via the additional supplementation with a dicarboxylic acid in the ratio of 1 part test solution to 4 parts dicarboxylic acid.
3) Clavulanic acid and cloxacillin were made up to a final concentration of 40µg/ml and 400µg/ml and mixed together in equal parts to yield the inhibitor mix.
4) Zinc sulphate and PEG 8000 were added to the inhibitor mix, to final concentrations of 1mM and 0.5% respectively.
5) A mixture containing 2mM polymixin, 4mM of a glycopeptide and 1.5mM of a polypeptide antibiotic were also dissolved in the solution containing the inhibitor mix
6) 2.8ml volumes solution containing the inhibitor mix, zinc sulphate, PEG 8000 and antibiotic mixture described in step 5 above were then freeze dried into pellets for 72hr

Subsequently, determining the presence of a carbapenemase was then carried out as described below:
(a) Reconstitute the pellet by adding 3.5ml of test solution from step 2 above.
(b) Allow the pellet to fully dissolve at room temperature for 1 minute and mix contents by gently vortexing for 10 seconds.
(c) Reconstituted solution should be yellow, if the solution is any other colour do not use.
(d) Dispense 500µl of reconstituted solution into microfuge tubes. One tube per test.
(e) Using a pure, fresh culture of the test organism, add a 1µl loopful of organism to the microfuge tube and mix well by vortexing for 10 seconds.
(f) Incubate at 35-37°C for 10 minutes and record the colour of the test solution

**Table 5: External and internal evaluation data showing high senstivity and specificity for the detection of carbapenemase producing Enterobacteriales, Pseudomonas and Acinetobacter spp isolates, with the present invention.**

| **Resistance Mechanisms** | | **Correctly Identified** (Carbapenemase Negative) | **Incorrectly Identified** (Carbapenemase Positive) |
|---|---|---|---|
| Non-Carbapenemase Producing Organisms | ESβL | 26 | 0 |
| | AmpC | 17 | 0 |
| | AmpC/PL | 6 | 0 |
| | Negative | 15 | 0 |
| | **Total** | **64** | **0** |
| | | | |

| **Resistance Mechanisms** | | **Correctly Identified** (Carbapenemase Positive) | **Incorrectly Identified** (Carbapenemase Negative) |
|---|---|---|---|
| Carbapenemase Producing Organisms | KPC | 14 | 1 |
| | MβL | 39 | 0 |
| | OXA-48 | 16 | 0 |
| | OXA-48 & ESβL | 1 | 0 |
| | OXA-95 & AmpC | 0 | 1 |
| | OXA-23 | 4 | 0 |
| | OXA-25 | 1 | 0 |
| | OXA-26 | 1 | 0 |
| | OXA-27 | 1 | 0 |
| | OXA-23 & OXA-51 | 2 | 0 |
| | OXA-58 | 1 | 0 |
| | OXA-23 & OXA-27 & OXA-51 | 1 | 0 |
| | **Total** | **81** | **2** |

| | |
|---|---|
| **Sensitivity** | **97.59%** |
| **Specificity** | **100.00%** |

110 Enterobacteriales were tested; of which 72 isolates were carbapenemase producers and 38 were non-carbapenemase producers. 57 of the tested isolates were *Klebsiella* spp [12 ESBLs, 5 AmpCs, 10 OXAs, 13 KPCs and 17 MβLs (6 NDMs, 6 IMPs and 5 VIMs)], 24 were *Escherichia coli* [8 ESBLs, 5 AmpCs, 5 OXAs, 1 KPC and 5 MβLs (2VIMs, 2NDMs and 1 IMP)], 23 were *Enterobacter* spp [6 ESBLs, 10 AmpCs, 2 OXAs, 4 MβLs (2 VIMs, 2 NDMs and 1 KPC)], 3 *Citrobacter* spp [all NDMs], 1 *Kluvyera* spp [an NDM producer] and 2 Salmonella spp isolates were tested; of which both were AmpC producers [non- carbapenemases]
- 23 *Pseudomonas* spp isolates were tested; of which 8 isolates were MβL carbapenemase producers comprising of 2 NDMs, 2 IMPs and 4 VIMs. 15 Pseudomonas isolates were carbapenemase non-producers [14 of these did not have any enzyme mediated mechanism, while one isolate was an AmpC producer].
- 14 *Acinetobacter* spp isolates were tested; of which 13 isolates were carbapenemase producers and one was a carbapenemase non-producer. Of the carbapenemase producers, there were 12 oxacillinase producing isolates [4 OXA-23s, 1 OXA-25, 1 OXA-26, 1 OXA-27, 1 OXA-58 and co-producers such as 2 OXA23 + OXA-51s, 1 OXA-23 + OXA-27 + OXA-51 and 1 OXA-95+AmpC]. One of the carbapenemase producing *Acinetobacter* isolates was an MβL producer.

As shown in Table 5, of the total 147 isolates tested, the present invention detected 81 of the 83 carbapenemase producing Enterobacteriales, *Pseudomonas* and *Acinetobacter* isolates. This correlates to a total sensitivity of 97.59% and 100% specificity for a wide range of Gram negative bacteria. Meanwhile a commercially available kit has been judged to be not suitable for use with Pseudomonas aeruginosa (Henrichs *et al.,* 2015), whilst another major competitor kit which is believed to employ the use of the chromogenic HMRZ-86 only showed a 64.9% sensitivity and 90% specificity for the detection of carbapenemase producing Enterobacteriaceae (Mancini *et al.,* 2017). Consequently, at this moment, the present invention is the sole rapid colorimetric carbapenemase detection method that is not only suitable for use with *Pseudomonas* and *Acinetobacter* spp isolates, but also extends to the Enterobacteriales; with very high sensitivity and specificity.

## Claims

1. A method for detecting the presence of carbapenemase-producing Enterobacteriales, *Pseudomonas* and *Acinetobacter* in a sample, comprising:
reacting a sample suspected of having carbapenemase-producing Enterobacteriales, *Pseudomonas* or *Acinetobacter* with a solution of (7R)-7-[(z)-2-(2-aminothiazol-4-yl)-(Z)-2(methoxyimino)acetamide]-3-(2,4-dinitrostyryl)-3-cephem-4 carboxylic acid) or a salt thereof, and
detecting a color change in the reaction medium when carbapenemase-producing Enterobacteriales, *Pseudomonas* or *Acinetobacter* are present in the test sample.

2. The method according to Claim 1, wherein the sample is a biological sample, optionally wherein the biological sample is selected from the group consisting of a biological sample obtained from a subject such as urine or from isolated cell culture.

3. The method according to any preceding claim, wherein the method further comprises a step of lysing the sample prior to, or simultaneously with, reaction with (7R)-7-[(z)-2-(2-aminothiazol-4-yl)-(Z)-2(methoxyimino)acetamide]-3-(2,4-dinitro styryl)-3-cephem-4 carboxylic acid) or a salt thereof.

4. The method according to claim 3, wherein sample lysis occurs in the presence of (7R)-7-[(z)-2-(2-aminothiazol-4-yl)-(Z)-2(methoxyimino)acetamide]-3-(2,4-dinitro styryl)-3-cephem-4 carboxylic acid) or a salt thereof, optionally wherein the sample lysis is facilitated by the combination of a polymyxin, glycopeptide and polypeptide antibiotic.

5. The method according to any preceding claim, wherein the reaction of the sample with ([(7R)-7-[(z)-2-(2-aminothiazol-4-yl)-(Z)-2(methoxyimino)acetamide]-3-(2,4-dinitro styryl)-3-cephem-4 carboxylic acid) or a salt thereof, is carried out over a period of time sufficient to observe a color change, preferably less than 1 hour, more preferably less than 30 minutes and most preferably between about 5 and about 20 minutes.

6. The method according to any preceding claim, wherein the reaction of the sample with ([(7R)-7-[(z)-2-(2-aminothiazol-4-yl)-(Z)-2(methoxyimino)acetamide]-3-(2,4-dinitro styryl)-3-cephem-4 carboxylic acid) or a salt thereof is carried out at a temperature between about 5 °C and about 40 °C, more preferably between about 20 °C and about 37 °C and most preferably at 35 °C .

7. The method according to any preceding claim, wherein the method further comprises an extended spectrum β-lactam (ESBL) inhibitor, a AmpC inhibitor or a mixture thereof.

8. The method according to any preceding claim wherein the ([(7R)-7-[(z)-2-(2-aminothiazol-4-yl)-(Z)-2(methoxyimino)acetamide]-3-(2,4-dinitro styryl)-3-cephem-4 carboxylic acid) or a salt thereof is used in combination with a carbapenem or penem alone, or in combination with an extended spectrum β-lactam (ESBL) inhibitor, a AmpC inhibitor or a mixture thereof.

9. The method according to any one of claims 7 and 8, wherein the extended spectrum inhibitor is clavulanic acid and the AmpC inhibitor is cloxacillin or a mixture thereof.

10. The method according to any preceding claim, wherein the inhibitor comprises at least one of clavulanic acid at a concentration of between about 30 µg/ml to about 50 µg/ml and cloxacillin at a concentration of between about 300 µg/ml to about 500 µg/ml.

11. The method according to any preceding claim, wherein the ([(7R)-7-[(z)-2-(2-aminothiazol-4-yl)-(Z)-2(methoxyimino)acetamide]-3-(2,4-dinitro styryl)-3-cephem-4 carboxylic acid) or a salt thereof is dissolved in a solution comprising at least one of an organic solvent, an organic acid and zinc sulphate.

12. The method according to Claim 8, wherein the organic solvent is a polar, aprotic solvent, preferably DMSO, and wherein the organic acid is a linear saturated dicarboxylic acid.

13. Use of ([(7R)-7-[(z)-2-(2-aminothiazol-4-yl)-(Z)-2(methoxyimino)acetamide]-3-(2,4-dinitro styryl)-3-cephem-4 carboxylic acid) or a salt thereof for detecting the presence of carbapenemase-producing Enterobacteriales, *Pseudomonas* and *Acinetobacter* in a sample.

14. Use of ([(7R)-7-[(z)-2-(2-aminothiazol-4-yl)-(Z)-2(methoxyimino)acetamide]-3-(2,4-dinitro styryl)-3-cephem-4 carboxylic acid) or a salt thereof according to the claim 13, wherein the ([(7R)-7-[(z)-2-(2-aminothiazol-4-yl)-(Z)-2(methoxyimino)acetamide]-3-(2,4-dinitro styryl)-3-cephem-4 carboxylic acid) or a salt thereof is used in conjunction with a carbapenem or penem alone, or in combination with an extended spectrum β-lactam (ESBL) inhibitor, an AmpC or a mixture thereof.

15. A kit for determining whether a microorganism produces a carbapenem-hydrolysing β-lactamase, comprising, a vial containing ([(7R)-7-[(z)-2-(2-aminothiazol-4-yl)-(Z)-2(methoxyimino)acetamide]-3-(2,4-dinitro styryl)-3-cephem-4 carboxylic acid) or a salt thereof, a microtitre plate having wells which are coated with ([(7R)-7-[(z)-2-(2-aminothiazol-4-yl)-(Z)-2(methoxyimino)acetamide]-3-(2,4-dinitrostyryl)-3-cephem-4 carboxylic acid) or a salt thereof, or an assay disk impregnated with HMRZ-98 ([(7R)-7-[(z)-2-(2-aminothiazol-4-yl)-(Z)-2(methoxyimino)acetamide]-3-(2,4-dinitro styryl)-3-cephem-4 carboxylic acid) or a salt thereof, and an extended spectrum β-lactam (ESBL) inhibitor, a AmpC inhibitor or a mixture thereof.

## Patentansprüche

1. Verfahren für den Nachweis der Gegenwart von Carbapenemase-produzierenden Enterobakterien *Pseudomonas* und *Acinetobacter* in einer Probe, das Folgendes umfasst:
Reagieren einer Probe, von der vermutet wird, dass sie Carbapenemaseproduzierende Enterobakterien *Pseudomonas* oder *Acinetobacter* aufweist, mit einer Lösung von (7R)-7-[(z)-2-(2-Aminothiazol-4-yl)-(Z)-2-(methoxyimino)acetamid]-3-(2,4-dinitrostyryl)-3-cephem-4-carbonsäure oder einem Salz davon und
Nachweisen einer Farbänderung in dem Reaktionsmedium, wenn Carbapenemaseproduzierende Enterobakterien *Pseudomonas* oder *Acinetobacter* in der Untersuchungsprobe vorliegen.

2. Verfahren nach Anspruch 1, wobei die Probe eine biologische Probe ist, optional wobei die biologische Probe aus der Gruppe ausgewählt ist, die aus einer biologischen Probe besteht, die von einem Subjekt erhalten wird, wie zum Beispiel Urin oder von isolierter Zellkultur.

3. Verfahren nach einem vorstehenden Anspruch, wobei das Verfahren weiter einen Schritt des Lysierens der Probe vor oder gleichzeitig mit der Reaktion mit (7R)-7-[(z)-2-(2-Aminothiazol-4-yl)-(Z)-2-(methoxyimino)acetamid]-3-(2,4-dinitrostyryl)-3-cephem-4-carbonsäure oder einem Salz davon umfasst.

4. Verfahren nach Anspruch 3, wobei die Probenlyse in Gegenwart von (7R)-7-[(z)-2-(2-Aminothiazol-4-yl)-(Z)-2-(methoxyimino)acetamid]-3-(2,4-dinitrostyryl)-3-cephem-4-carbonsäure oder einem Salz davon erfolgt, optional wobei die Probenlyse durch die Kombination eines Polymyxin-, Glycopeptid- und Polypeptid-Antibiotikums unterstützt wird.

5. Verfahren nach einem vorstehenden Anspruch, wobei die Reaktion der Probe mit (7R)-7-[(z)-2-(2-Aminothiazol-4-yl)-(Z)-2-(methoxyimino)acetamid]-3-(2,4-dinitrostyryl)-3-cephem-4-carbonsäure oder einem Salz davon über eine Zeitdauer durchgeführt wird, die ausreicht, um eine Farbänderung zu beobachten, bevorzugt von weniger als 1 Stunde, bevorzugter von weniger als 30 Minuten und am bevorzugtesten zwischen etwa 5 und etwa 20 Minuten.

6. Verfahren nach einem vorstehenden Anspruch, wobei die Reaktion der Probe mit (7R)-7-[(z)-2-(2-Aminothiazol-4-yl)-(Z)-2-(methoxyimino)acetamid]-3-(2,4-dinitrostyryl)-3-cephem-4-carbonsäure oder einem Salz davon bei einer Temperatur von zwischen etwa 5 °C und etwa 40 °C, bevorzugter von zwischen etwa 20 °C und etwa 37 °C und am bevorzugtesten bei 35 °C durchgeführt wird.

7. Verfahren nach einem vorstehenden Anspruch, wobei das Verfahren weiter einen β-Lactam-Inhibitor mit erweitertem Spektrum (ESBL inhibitor = extended spectrum β-lactam inhibitor), einen AmpC-Inhibitor oder eine Mischung davon umfasst.

8. Verfahren nach einem vorstehenden Anspruch, wobei die (7R)-7-[(z)-2-(2-Aminothiazol-4-yl)-(Z)-2-(methoxyimino)acetamid]-3-(2,4-dinitrostyryl)-3-cephem-4-carbonsäure oder ein Salz davon in Kombination mit einem Carbapenem oder Penem allein oder in Kombination mit einem β-Lactam-Inhibitor mit erweitertem Spektrum (ESBL-Inhibitor), einem AmpC-Inhibitor oder einer Mischung davon verwendet wird.

9. Verfahren nach einem der Ansprüche 7 und 8, wobei der Inhibitor mit erweitertem Spektrum Clavulansäure ist und der AmpC-Inhibitor Cloxacillin ist, oder eine Mischung davon.

10. Verfahren nach einem vorstehenden Anspruch, wobei der Inhibitor mindestens eines von Clavulansäure in einer Konzentration von zwischen etwa 30 µg/ml bis etwa 50 µg/ml und Cloxacillin in einer Konzentration von zwischen etwa 300 µg/ml bis etwa 500 µg/ml umfasst.

11. Verfahren nach einem vorstehenden Anspruch, wobei die (7R)-7-[(z)-2-(2-Aminothiazol-4-yl)-(Z)-2-(methoxyimino)acetamid]-3-(2,4-dinitrostyryl)-3-cephem-4-carbonsäure oder ein Salz davon in einer Lösung gelöst wird, die mindestens eines von einem organischen Lösungsmittel, einer organischen Säure und Zinksulfat umfasst.

12. Verfahren nach Anspruch 8, wobei das organische Lösungsmittel ein polares, aprotisches Lösungsmittel ist, bevorzugt DMSO, und wobei die organische Säure eine lineare gesättigte Dicarbonsäure ist.

13. Verwendung von (7R)-7-[(z)-2-(2-Aminothiazol-4-yl)-(Z)-2-(methoxyimino)acetamid]-3-(2,4-dinitrostyryl)-3-cephem-4-carbonsäure oder eines Salzes davon für den Nachweis der Gegenwart von Carbapenemase-produzierenden Enterobakterien *Pseudomonas* und *Acinetobacter* in einer Probe.

14. Verwendung von (7R)-7-[(z)-2-(2-Aminothiazol-4-yl)-(Z)-2-(methoxyimino)acetamid]-3-(2,4-dinitrostyryl)-3-cephem-4-carbonsäure oder eines Salzes davon nach Anspruch 13, wobei die (7R)-7-[(z)-2-(2-Aminothiazol-4-yl)-(Z)-2-(methoxyimino)acetamid]-3-(2,4-dinitrostyryl)-3-cephem-4-carbonsäure oder ein Salz davon in Verbindung mit einem Carbapenem oder Penem allein oder in Kombination mit einem β-Lactam-Inhibitor mit erweitertem Spektrum (ESBL-Inhibitor), einem AmpC- oder einer Mischung davon verwendet wird.

15. Kit für die Bestimmung, ob ein Mikroorganismus eine Carbapenemhydrolysierende β-Lactamase produziert, der Folgendes umfasst: ein Fläschchen, das (7R)-7-[(z)-2-(2-Aminothiazol-4-yl)-(Z)-2-(methoxyimino)acetamid]-3-(2,4-dinitrostyryl)-3-cephem-4-carbonsäure oder ein Salz davon enthält, eine Mikrotiterplatte, die Wells aufweist, die mit (7R)-7-[(z)-2-(2-Aminothiazol-4-yl)-(Z)-2-(methoxyimino)acetamid]-3-(2,4-dinitrostyryl)-3-cephem-4-carbonsäure oder einem Salz davon beschichtet sind, oder eine Assay-Platte, die mit HMRZ-98 ((7R)-7-[(z)-2-(2-Aminothiazol-4-yl)-(Z)-2-(methoxyimino)acetamid]-3-(2,4-dinitrostyryl)-3-cephem-4-carbonsäure) oder einem Salz davon imprägniert ist, und einen β-Lactam-Inhibitor mit erweitertem Spektrum (ESBL-Inhibitor), einen AmpC-Inhibitor oder eine Mischung davon.

## Revendications

1. Méthode de détection de la présence d'Entérobactéries productrices de carbapénémase, *Pseudomonas* et *Acinetobacter,* dans un échantillon, comprenant :
la réaction d'un échantillon suspecté comme contenant des Entérobactéries productrices de carbapénémase, *Pseudomonas* ou *Acinetobacter,* avec une solution d'acide (7R)-7-[(z)-2-(2-aminothiazol-4-yl)-(Z)-2-(méthoxyimino)acétamide]-3-(2,4-dinitrostyryl)-3-céphem-4-carboxylique ou d'un sel de celui-ci, et
la détection d'un changement de couleur dans le milieu réactionnel lorsque des Entérobactéries productrices de carbapénémase, *Pseudomonas* ou *Acinetobacter,* sont présentes dans l'échantillon à tester.

2. Méthode selon la revendication 1, dans laquelle l'échantillon est un échantillon biologique, éventuellement où l'échantillon biologique est choisi dans le groupe constitué par un échantillon biologique obtenu à partir d'un sujet, tel que l'urine, ou à partir d'une culture cellulaire isolée.

3. Méthode selon l'une quelconque des revendications précédentes, la méthode comprenant en outre une étape de lyse de l'échantillon préalablement à, ou simultanément avec, la réaction avec l'acide (7R)-7-[(z)-2-(2-aminothiazol-4-yl)-(Z)-2-(méthoxyimino)acétamide]-3-(2,4-dinitrostyryl)-3-céphem-4-carboxylique ou un sel de celui-ci.

4. Méthode selon la revendication 3, dans laquelle la lyse de l'échantillon se produit en présence d'acide (7R)-7-[(z)-2-(2-aminothiazol-4-yl)-(Z)-2-(méthoxyimino)-acétamide]-3-(2,4-dinitrostyryl)-3-céphem-4-carboxylique ou d'un sel de celui-ci, éventuellement où la lyse de l'échantillon est facilitée par la combinaison d'un antibiotique de type polymyxine, glycopeptide et polypeptide.

5. Méthode selon l'une quelconque des revendications précédentes, dans laquelle la réaction de l'échantillon avec l'acide (7R)-7-[(z)-2-(2-aminothiazol-4-yl)-(Z)-2-(méthoxyimino)acétamide]-3-(2,4-dinitrostyryl)-3-céphem-4-carboxylique ou un sel de celui-ci est effectuée sur une période de temps suffisante pour observer un changement de couleur, préférablement inférieure à 1 heure, plus préférablement inférieure à 30 minutes et tout préférablement comprise entre environ 5 et environ 20 minutes.

6. Méthode selon l'une quelconque des revendications précédentes, dans laquelle la réaction de l'échantillon avec l'acide (7R)-7-[(z)-2-(2-aminothiazol-4-yl)-(Z)-2-(méthoxyimino)acétamide]-3-(2,4-dinitrostyryl)-3-céphem-4-carboxylique ou un sel de celui-ci est effectuée à une température comprise entre environ 5°C et environ 40°C, plus préférablement entre environ 20°C et environ 37°C et tout préférablement à 35°C.

7. Méthode selon l'une quelconque des revendications précédentes, la méthode comprenant en outre un inhibiteur de β-lactamase à spectre élargi (BLSE), un inhibiteur d'AMPc ou un mélange de ceux-ci.

8. Méthode selon l'une quelconque des revendications précédentes, dans laquelle l'acide (7R)-7-[(z)-2-(2-aminothiazol-4-yl)-(Z)-2-(méthoxyimino)acétamide]-3-(2,4-dinitrostyryl)-3-céphem-4-carboxylique ou un sel de celui-ci est utilisé en combinaison avec un carbapénème ou un pénème seul, ou en combinaison avec un inhibiteur de β-lactamase à spectre élargi (BLSE), un inhibiteur d'AMPc ou un mélange de ceux-ci.

9. Méthode selon l'une quelconque des revendications 7 et 8, dans laquelle l'inhibiteur à spectre élargi est l'acide clavulanique et l'inhibiteur d'AMPc est la cloxacilline ou un mélange de ceux-ci.

10. Méthode selon l'une quelconque des revendications précédentes, dans laquelle l'inhibiteur comprend au moins l'un parmi l'acide clavulanique selon une concentration allant d'environ 30 µg/ml à environ 50 µg/ml et la cloxacilline selon une concentration allant d'environ 300 µg/ml à environ 500 µg/ml.

11. Méthode selon l'une quelconque des revendications précédentes, dans laquelle l'acide (7R)-7-[(z)-2-(2-aminothiazol-4-yl)-(Z)-2-(méthoxyimino)acétamide]-3-(2,4-dinitrostyryl)-3-céphem-4-carboxylique ou un sel de celui-ci est dissous dans une solution comprenant au moins l'un parmi un solvant organique, un acide organique et du sulfate de zinc.

12. Méthode selon la revendication 8, dans laquelle le solvant organique est un solvant aprotique polaire, préférablement le DMSO, et où l'acide organique est un acide dicarboxylique saturé linéaire.

13. Utilisation de l'acide (7R)-7-[(z)-2-(2-aminothiazol-4-yl)-(Z)-2-(méthoxyimino)acétamide]-3-(2,4-dinitrostyryl)-3-céphem-4-carboxylique ou d'un sel de celui-ci pour la détection de la présence d'Entérobactéries productrices de carbapénémase, *Pseudomonas* et *Acinetobacter,* dans un échantillon.

14. Utilisation de l'acide (7R)-7-[(z)-2-(2-aminothiazol-4-yl)-(Z)-2-(méthoxyimino)acétamide]-3-(2,4-dinitrostyryl)-3-céphem-4-carboxylique ou d'un sel de celui-ci selon la revendication 13, dans laquelle l'acide (7R)-7-[(z)-2-(2-aminothiazol-4-yl)-(Z)-2-(méthoxyimino)acétamide]-3-(2,4-dinitrostyryl)-3-céphem-4-carboxylique ou un sel de celui-ci est utilisé en combinaison avec un carbapénème ou un pénème seul, ou en combinaison avec un inhibiteur de β-lactamase à spectre élargi (BLSE), une AMPc ou un mélange de ceux-ci.

15. Kit destiné à déterminer si un microorganisme produit une β-lactamase hydrolysant les carbapénèmes, comprenant un flacon contenant de l'acide (7R)-7-[(z)-2-(2-aminothiazol-4-yl)-(Z)-2-(méthoxyimino)acétamide]-3-(2,4-dinitrostyryl)-3-céphem-4-carboxylique ou un sel de celui-ci, une plaque de microtitration ayant des puits qui sont revêtus d'acide (7R)-7-[(z)-2-(2-aminothiazol-4-yl)-(Z)-2-(méthoxyimino)-acétamide]-3-(2,4-dinitrostyryl)-3-céphem-4-carboxylique ou d'un sel de celui-ci, ou un disque d'essai imprégné par HMRZ-98 (acide (7R)-7-[(z)-2-(2-aminothiazol-4-yl)-(Z)-2-(méthoxyimino)acétamide]-3-(2,4-dinitrostyryl)-3-céphem-4-carboxylique) ou un sel de celui-ci, et un inhibiteur de β-lactamase à spectre élargi (BLSE), un inhibiteur d'AMPc ou un mélange de ceux-ci.
